(19) Europäisches Patentamt
European Patent Office
Office européen des brevets

(11) Veröffentlichungsnummer : **0 037 479**
**B1**

(12) # EUROPÄISCHE PATENTSCHRIFT

(45) Veröffentlichungstag der Patentschrift : **30.03.83**

(51) Int. Cl.$^3$ : **C 07 D233/96**

(21) Anmeldenummer : **81101881.1**

(22) Anmeldetag : **13.03.81**

(54) **Verfahren zur Herstellung von 5-Arylidenhydantoinen (B).**

(30) Priorität : **09.04.80 DE 3013647**

(43) Veröffentlichungstag der Anmeldung : **14.10.81 Patentblatt 81/41**

(45) Bekanntmachung des Hinweises auf die Patenterteilung : **30.03.83 Patentblatt 83/13**

(84) Benannte Vertragsstaaten : **BE CH FR GB IT LI NL**

(56) Entgegenhaltungen :
**DE B 1 038 050**
**CHEMICAL ABSTRACTS, vol. 82, no. 21, 26. Mai 1975, Seite 630 Zusammenfassung 140139v**

(73) Patentinhaber : **Degussa Aktiengesellschaft**
**Weissfrauenstrasse 9**
**D-6000 Frankfurt am Main 1 (DE)**

(72) Erfinder : **Kleemann, Axel, Dr. Dipl.-Chem.**
**Greifenhagenstrasse 9**
**D-6450 Hanau 9 (DE)**
Erfinder : **Lüssling, Theodor, Dr. Dipl.-Chem.**
**Zum Purren 5**
**D-7750 Konstanz (DE)**
Erfinder : **Pfeifer, Wolf, Dr. Dipl.-Chem.**
**Stettiner Strasse 4**
**D-5040 Brühl (DE)**
Erfinder : **Scherberich, Paul, Dr. Dipl.-Chem.**
**Königsberger Strasse 8**
**D-7750 Konstanz (DE)**

**EP 0 037 479 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 5-Arylidenhydantoinen (B)

## Beschreibung

Gegenstand der Erfindung ist ein Verfahren zur Herstellung von im aromatischen Kern unsubstituierten oder substituierten 5-Arylidenhydantoinen durch Kondensation von entsprechenden unsubstituierten oder substituierten aromatischen Aldehyden mit Hydantoin in Gegenwart eines Carbonsäuresalzes.

5-Arylidenhydantoine sind wichtige Zwischenprodukte für die Herstellung von Phenylalanin und im aromatischen Kern substituierten Phenylalaninen.

Es ist bereits bekannt, 5-(3',4'-Dioxybenzyliden)-hydantoin durch Kondensation von Protocatechualdehyd mit Hydantoin in durch Ammoniumchlorid gepufferter Natronlauge herzustellen (DE-PS 1 038 050).

Es ist ferner bereits bekannt, 5-(3',4'-Dimethoxybenzyliden)-hydantoin durch Kondensation von 3,4-Dimethoxybenzaldehyd mit Hydantoin in wässriger Lösung und in Gegenwart von Diäthanolamin oder Ammoniak herzustellen (Chem. Abstr. 82 (21), *1975*, Referat 140 139v).

Es ist auch schon bekannt, im aromatischen Kern unsubstituierte oder substituierte 5-Arylidenhydantoine durch Kondensation der entsprechenden aromatischen Aldehyde mit Hydantoin in Gegenwart von Monoäthanolamin herzustellen (Französische Patentanmeldung 73.21868 — Veröffentlichungs nummer 2 189 040).

Schließlich ist es auch bereits bekannt, 5-Arylidenhydantoine durch Kondensation von Benzaldehyd oder substituierten aromatischen Aldehyden mit Hydantoin in Gegenwart von Essigsäure und von wasserfreiem Natriumacetat herzustellen (J. Amer. Chem. Soc. *45*, Seiten 368 bis 383 (1911)). Bei der Umsetzung von Benzaldehyd betragen die Ausbeuten beispielsweise 70 bis 80 %, bezogen auf eingesetztes Hydantoin. Zur Erzielung dieser Ausbeuten ist jedoch der Einsatz relativ grosser Mengen an wasserfreiem Natriumacetat erforderlich. Wird die Menge des Natriumacetats vermindert, so geht die Ausbeute drastisch zurück.

Das erfindungsgemässe Verfahren ist nun dadurch gekennzeichnet, dass man die Kondensation in Gegenwart mindestens eines Ammoniumsalzes einer aliphatischen oder aromatischen Carbonsäure vornimmt.

Das erfindungsgemässe Verfahren ergibt überraschenderweise auch schon dann höhere Ausbeuten an dem gewünschten Hydantoin als das bekannte Verfahren, wenn die Ammoniumsalze in erheblich geringerer Menge eingesetzt werden.

Das erfindungsgemässe Verfahren eignet sich besonders zur Herstellung von 5-Arylidenhydantoinen der allgemeinen Formel

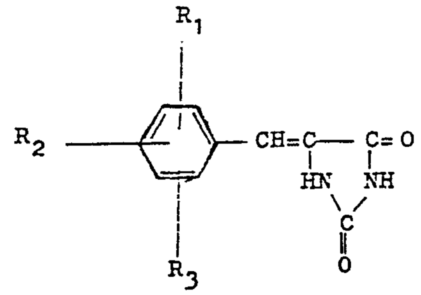

(I)

in der $R_1$, $R_2$ und $R_3$ gleich oder verschieden sind und jeweils Wasserstoff, Halogen, eine Hydroxylgruppe, eine Nitrogruppe, eine Aminogruppe, eine unverzweigte oder verzweigte Alkyl- oder Alkenylgruppe, eine Cycloalkylgruppe, eine Cycloalkenylgruppe, eine Arylgruppe, eine Aralkylgruppe, eine Alkarylgruppe, eine Alkoxygruppe, eine Alkylthiogruppe, eine Acyloxygruppe, eine Acylthiogruppe, eine Mono- oder Dialkylaminogruppe oder eine Acylaminogruppe bedeuten. Vorzugsweise enthalten die Alkylgruppen 1 bis 6, insbesondere 1 bis 3, Kohlenstoffatome, die Alkenylgruppen 2 bis 6, insbesondere 2 bis 3, Kohlenstoffatome, die Cycloalkyl- und Cycloalkenylgruppen 3 bis 8, vorzugsweise 3 bis 6, Kohlenstoffatome. Gegebenenfalls kann in den Cycloalkyl- und Cycloalkenylgruppen auch einmal oder mehrmals $—CH_2—$ durch —O—, —S— oder —NH— beziehungsweise —CH = durch —N = ersetzt sein, so dass entsprechende heterocyclische Ringe mit 3 bis 8, insbesondere 3 bis 6, Ringgliedern vorliegen. Vorzugsweise enthalten die Aralkyl- und die Alkarylgruppen 1 bis 6, insbesondere 1 bis 3, Kohlenstoffatome in der Alkylen- beziehungsweise Alkylgruppe. Die Alkoxy-, Alkylthio-, Acyloxy-, Acylthio-, Mono- oder Dialkylamino- und Acylaminogruppen enthalten vorzugsweise 1 bis 6, insbesondere 1 bis 3, Kohlenstoffatome in den Alkyl- beziehungsweise Acylgruppen. Gegebenenfalls können auch zwei der Reste $R_1$ bis $R_3$ zusammen eine Alkylen- oder Alkenylengruppe mit 1 bis 6, insbesondere 1 bis 4, Kohlenstoffatomen bilden, wobei auch in diesem Fall einmal oder mehrmals $—CH_2—$ durch —O—, —S— oder —NH— beziehungsweise —CH = durch —N = ersetzt sein kann.

Eingesetzt werden können demnach aromatische Aldehyde der allgemeinen Formel

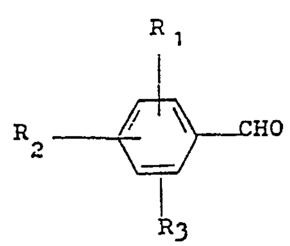

(II)

in der $R_1$, $R_2$ und $R_3$ die vorstehend angegebene Bedeutung haben. Solche Aldehyde sind beispielsweise Benzaldehyd, Tolylaldehyd, 4-Isopro-

pylbenzaldehyd, 4-Hydroxybenzaldehyd, 3,4,5-Trimethoxybenzaldehyd, 3-Brom-4-methoxybenzaldehyd, 3-4-Methylendioxybenzaldehyd, 2-Hydroxy-4-nitrobenzaldehyd, 4,5-Dimethoxy-2-nitrobenzaldehyd, Salicylaldehyd, Vanillin, 4-Phenylbenzaldehyd, 4-Benzylbenzaldehyd, 4-Fluorbenzaldehyd, 4-Dimethylaminobenzaldehyd, 4-Acetoxybenzaldehyd, 4-Acetaminobenzaldehyd, 4-Methylthiobenzaldehyd und 3,5-Dichlor-4-hydroxybenzaldehyd.

Die Kondensation mit dem Hydantoin erfolgt in Gegenwart von Ammoniumsalzen aliphatischer und/oder aromatischer Carbonsäuren, wie beispielsweise Ameisensäure, Essigsäure, Propionsäure, Buttersäure, 2-Methylbuttersäure, Valeriansäure, Isovaleriansäure, 2-Chloressigsäure, 2-Hydroxyessigsäure, Benzoesäure, Mandelsäure, Phenylessigsäure, Zimtsäure, Phenylpropionsäure oder Crotonsäure, oder von Gemischen solcher Carbonsäuren.

Die Ammoniumsalze können als solche eingesetzt oder aus der betreffenden Carbonsäure und Ammoniak im Reaktionsgemisch erzeugt werden. Es kann vorteilhaft sein, die Kondensation in einem Lösungsmittel durchzuführen. Als solche kommen überschüssige Carbonsäure, aliphatische oder aromatische Alkohole, aliphatische oder aromatische Äther, aliphatische oder aromatische Kohlenwasserstoffe, halogenierte aliphatische oder aromatische Kohlenwasserstoffe und deren Gemische in Frage. Beispielsweise können n-Butanol, tert-Butanol, Toluol, Benzylalkohol, Xylol, Chlorbenzol und deren Gemische verwendet werden.

Im allgemeinen erfolgt die Kondensation bei Temperaturen etwa zwischen 80 und 200 °C, insbesondere bei Temperaturen zwischen 100 °C und der Siedetemperatur des Gemisches. Der Druck kann weitgehend beliebig gewählt werden. Es kann also bei Normaldruck wie auch bei niedrigerem oder höherem Druck gearbeitet werden. Obwohl es im allgemeinen vorteilhaft ist, bei etwa Normaldruck zu arbeiten, kann wegen der Flüchtigkeit der Substanzen erhöhter Druck erforderlich sein.

Das Mengenverhältnis von Aldehyd zu Hydantoin kann sowohl stöchiometrisch als auch unter- oder überstöchiometrisch gewählt werden. Im allgemeinen ist es vorteilhaft, je Mol Hydantoin etwa 1 bis 2 Mol, insbesondere 1,2 bis 1,5 Mol, des Aldehyds einzusetzen.

Je Mol Hydantoin werden zweckmässigerweise wenigstens 0,2 Mol, vorzugsweise 0,5 bis 2,0 Mol, insbesondere 0,8 bis 1,2 Mol, der Ammoniumsalze eingesetzt.

Es kann weiterhin vorteilhaft sein, das bei der Kondensation entstehende Wasser während der Reaktion durch Destillation abzutrennen.

Beispiel 1

Ein Gemisch aus 100 g (1,0 Mol) Hydantoin, 117 g (1,1 Mol) Benzaldehyd, 77 g (1,0 Mol) Ammoniumacetat und 240 g (4,0 Mol) Essigsäure wurde 4 Stunden lang auf Rückflusstemperatur gehalten. Beim Abkühlen auf Raumtemperatur schied sich das 5-Benzylidenhydantoin ab. Die Ausbeute betrug 179 g, entsprechend 95 % der Theorie, bezogen auf Hydantoin. Das Produkt hatte einen Schmelzpunkt von 219-220 °C und war, wie durch Dünnschichtchromatographie festgestellt wurde, einheitlich.

Vergleichsversuch 1

Es wurde wie nach Beispiel 1 verfahren, jedoch wurden anstelle von Ammoniumacetat 82 g (1,0 Mol) Natriumacetat eingesetzt. Es wurden 86, 5 g, entsprechend 46 % der Theorie, bezogen auf Hydantoin, an 5-Benzylidenhydantoin erhalten.

Vergleichsversuch 2

Es wurde wie nach Beispiel 1 verfahren, jedoch wurden anstelle von Ammoniumacetat 98 g (1,0 Mol) Kaliumacetat eingesetzt. Es wurden 79 g, entsprechend 42 % der Theorie, bezogen auf Hydantoin, an 5-Benzylidenhydantoin erhalten.

Beispiel 2

Ein Gemisch aus 100 g (1,0 Mol) Hydantoin, 106 g (1,0 Mol) Benzaldehyd, 63 g (1,0 Mol) Ammoniumformiat und 230 g (5,0 Mol) Ameisensäure wurde 3 Stunden lang auf Rückflusstemperatur gehalten. Nach dem Abkühlen wurden 173 g 5-Benzylidenhydantoin, entsprechend 92 % der Theorie, bezogen auf Hydantoin, erhalten.

Beispiel 3

Einem Gemisch aus 100 g (1,0 Mol) Hydantoin, 106 g (1,0 Mol) Benzaldehyd und 370 g (5,0 Mol) Propionsäure wurden 17 g (1,0 Mol) Ammoniak zugesetzt. Es wurde anschliessend 3 Stunden lang auf Rückflusstemperatur gehalten. Nach dem Abkühlen wurden 178 g 5-Benzylidenhydantoin, entsprechend 95 % der Theorie, bezogen auf Hydantoin, erhalten.

Beispiel 4

Ein Gemisch aus 100 g (1,0 Mol) Hydantoin, 151 g (1,0 Mol) 4-Nitrobenzaldehyd, 77 g (1,0 Mol) Ammoniumacetat, 120 g (2,0 Mol) Essigsäure und 200 ml n-Butanol wurde 3 Stunden lang auf Rückflusstemperatur gehalten. Nach dem Abkühlen wurden 219 g 5-(4'-Nitrobenzyliden)-hydantoin, entsprechend 94 % der Theorie, bezogen auf Hydantoin, erhalten. Das Produkt hatte einen Schmelzpunkt von 253-254 °C und war dünnschichtchromatographisch einheitlich.

Beispiel 5

Es wurde wie nach Beispiel 1 verfahren, jedoch wurden 119 g (1,1 Mol) 4-Methoxybenzaldehyd eingesetzt. Die Ausbeute an 5-(4'-Methoxybenzyliden)-hydantoin betrug 207 g, entsprechend 95 %

der Theorie, bezogen auf Hydantoin. Das Produkt hatte einen Schmelzpunkt von 243-244 °C.

Beispiel 6

Es wurde wie nach Beispiel 1 verfahren, jedoch wurden 169 g (1,2 Mol) 2-Chlorbenzaldehyd eingesetzt. Die Ausbeute an 5-(2'-Chlorbenzyliden)-hydantoin betrug 200 g, entsprechend 90 % der Theorie, bezogen auf Hydantoin.

Beispiel 7

Es wurde wie nach Beispiel 1 verfahren, jedoch wurden 150 g (1,0 Mol) 3,4-Methylendioxybenzaldehyd eingesetzt. Die Ausbeute an 5-(3',4'-Methylendioxybenzyliden)-hydantoin betrug 207 g, entsprechend 89 % der Theorie, bezogen auf Hydantoin.

Beispiel 8

Es wurde wie nach Beispiel 1 verfahren, jedoch wurden 122 g (1,0 Mol) 4-Hydroxybenzaldehyd eingesetzt. Die Ausbeute an 5-(4'-Hydroxybenzyliden)-hydantoin betrug 184 g, entsprechend 90 % der Theorie, bezogen auf Hydantoin. Das Produkt hatte einen Schmelzpunkt von 314-316 °C und war dünnschichtchromatographisch einheitlich.

**Ansprüche**

1. Verfahren zur Herstellung von im aromatischen Kern unsubstituierten oder substituierten 5-Arylidenhydantoinen durch Kondensation von entsprechenden unsubstituierten oder substituierten aromatischen Aldehyden mit Hydantoin in Gegenwart eines Carbonsäuresalzes, dadurch gekennzeichnet, dass man die Kondensation in Gegenwart mindestens eines Ammoniumsalzes einer aliphatischen oder aromatischen Carbonsäure vornimmt.

2. Verfahren nach Anspruch 1, dadurch gekennzeichnet, daß man das Ammoniumsalz in einer Menge von 0,5 bis 2,0 Mol pro Mol umzusetzenden Hydantoins einsetzt.

**Claims**

1. A process for the production of 5-arylidene hydantoins which are substituted or unsubstituted in the aromatic nucleus, by the condensation of corresponding substituted or unsubstituted aromatic aldehydes with hydantoin in the presence of a carboxylic acid salt, characterised in that the condensation is carried out in the presence of at least one ammonium salt of an aliphatic or aromatic carboxylic acid.

2. A process according to claim 1, characterised in that the ammonium salt is used in a quantity of from 0.5 to 2.0 mols per mol of hydantoin to be reacted.

**Revendications**

1. Procédé d'obtention de 5-arylidène-hydantoïnes, substituées ou non sur le noyau aromatique, par condensation de l'aldéhyde aromatique correspondant, substitué ou non, et d'hydantoïne en présence d'un sel d'acide carboxylique, procédé caractérisé en ce que la condensation est effectuée en présence d'au moins un sel d'ammonium d'un acide carboxylique aliphatique ou aromatique.

2. Procédé selon la revendication 1, caractérisé en ce que l'on utilise le sel d'ammonium dans une proportion de 0,5 à 2 moles par mole d'hydantoïne à condenser.